# EUROPÄISCHE PATENTSCHRIFT

(11) **EP 3 297 574 B1**
(45) Veröffentlichungstag und Bekanntmachung des Hinweises auf die Patenterteilung: **07.08.2019**
(21) Anmeldenummer: 16728844.8
(22) Anmeldetag: 20.05.2016
(51) Int. Cl.: A61F 2/16

(54) **KAPSELSPANNRING**
CAPSULE TENSIONING RING
ANNEAU DE TENSION CAPSULAIRE

(30) Priorität: 21.05.2015 CH 7142015
(43) Veröffentlichungstag der Anmeldung: 28.03.2018
(73) Patentinhaber: Feusi, Marco, 6204 Sempach (CH)
(72) Erfinder: Feusi, Marco, 6204 Sempach (CH)
(74) Vertreter: Hoffmann Eitle
(86) Internationale Anmeldenummer: PCT/EP2016/061424
(87) Internationale Veröffentlichungsnummer: WO 2016/185017

(56) Entgegenhaltungen:
- WO-A1-91/15166
- WO-A1-2008/108523
- WO-A1-2014/134302
- CN-A- 102 271 623
- US-A1- 2003 149 480
- US-A1- 2009 005 865

## Beschreibung

### TECHNISCHES GEBIET

Die vorliegende Erfindung betrifft einen Kapselspannring zum Implantieren in einem Augenkapselsack.

### STAND DER TECHNIK

Nach einer Kataraktoperation oder einer ähnlichen Augenoperation, bei der die natürliche Linse des Auges durch eine Intraokularlinse ersetzt wird, besteht grundsätzlich das Problem, dass der Kapselsack, in den die Intraokularlinse implantiert wird, schrumpft oder in sich zusammenfällt. Um dies zu verhindern, gibt es im Stand der Technik bereits Kapselspannringe, die in den Augenkapselsack implantiert werden und diesen stützen sollen. Gleichzeitig dienen diese Kapselspannringe manchmal dazu, die Intraokularlinse zu fixieren, wobei sich die Intraokularlinse am Kapselspannring abstützt.

Bekannte Kapselspannringe sind aus einem weitestgehend unflexiblen Material gefertigt, was einerseits dazu führt, dass bei der Implantation ein relativ großer Einschnitt erzeugt werden muss, um den Spannring in den Kapselsack einführen zu können. Andererseits erschwert das unflexible Material auch ein Entfernen des Spannrings, weil hierfür ebenfalls ein verhältnismäßig großer Schnitt in den Kapselsack eingebracht werden muss.

Aus DE 10 2004 027 236 A1 ist ein Kapselspannring bekannt, der mehrere falt- und/oder knickbare Segmente sowie steife Segmente aufweist, die in Umfangsrichtung jeweils abwechselnd angeordnet sind. Bei diesem Kapselspannring besteht insbesondre das Problem, dass er nur schwer aus dem Kapselsack entnommen werden kann, nachdem er einmal implantiert wurde, da die Konstruktion der faltbaren Segmente nicht zu Vorteilen beim Herausnehmen des Kapselspannrings führt.

DE 298 01 281 U1 offenbart einen intraokularen Ring, der mit Halterungen versehen ist, um Linsen eines Linsensystems über Haptiken zu fixieren. Dieser Ring ist steif ausgebildet und lässt sich daher schwer in den Kapselsack implantieren oder aus diesem herausnehmen.

US 2003/149480 A1 offenbart einen implantierbaren Kapselformkörper mit einer adaptiven Optik und zwei Fluidkammern, zwischen denen ein Fluid strömen kann, um die Optik zum Erhöhen und Verringern ihrer Stärke anzupassen.

### DARSTELLUNG DER ERFINDUNG

Vor dem Hintergrund des Standes der Technik besteht eine Aufgabe der vorliegenden Erfindung darin, einen Kapselspannring des obigen technischen Gebiets bereitzustellen, der sowohl ein Schrumpfen oder Zusammenfallen des Kapselsacks verhindert und dabei eine Intraokularlinse sicher und positionsgenau in dem Kapselsack halten kann, als auch bezüglich des Implantierens und des Herausnehmens des Kapselspannrings aus dem Kapselsack verbessert ist.

Die Lösung dieser Aufgabe wird durch den Kapselspannring nach Anspruch 1 gegeben. Vorteilhafte Ausführungsformen sind Gegenstand der Unteransprüche.

Ein erfindungsgemäßer Kapselspannring zum Implantieren in einem Augenkapselsack weist zwei Kammern auf, die sich jeweils entlang einer Umfangsrichtung des Kapselspannrings erstrecken. Dabei sind die Kammern dazu ausgebildet, je ein Fluid aufzunehmen. Ferner weist der Kapselspannring eine sich entlang der Umfangsrichtung erstreckende Nut auf, um dadurch eine Intraokularlinse zu fixieren. Diese "Nut" oder "Ausnehmung" lässt sich besonders effizient dadurch erzeugen, dass die beiden Kammern des Kapselspannrings jeweils durch einen entlang einer Ringform verlaufenden Schlauch mit kreisförmigem Querschnitt gebildet sind, die parallel zueinander aneinander angebracht sind. Die hierdurch entstehende Taille (gesehen im Querschnitt), die sich entlang der Verbindungsstelle der beiden insgesamt torusförmigen Schläuche bildet, definiert dabei an einer Innenseite des Kapselspannrings eine Nut mit einem im Wesentlichen V-förmigen Querschnitt. Mit anderen Worten verlaufen die Seitenwände der Nut im Wesentlichen V-förmig aufeinander zu. Diese Konfiguration der Nut erleichtert es besonders, eine Intraokularlinse in der Nut zu fixieren, weil die üblichen Haptiken solcher Intraokularlinsen sich in der im Wesentlichen V-förmigen Nut selbst zentrieren.

Die beiden Kammern sind vorzugweise jeweils zumindest teilweise torusförmig, können jedoch auch andere als kreisförmige oder elliptische Querschnitte, gesehen senkrecht zur Ringebene, d.h. der Ebene, in der sich der Ring insgesamt hauptsächlich erstreckt, aufweisen. Beispielsweise ist es möglich, dass die Kammern einen polygonalen Querschnitt senkrecht zur Ringebene aufweisen. Die Torusform erweist sich jedoch als vorteilhaft, weil hierdurch erstens effizient eine vorteilhafte Form der Nut entstehen kann und zweitens durch die abgerundeten Oberflächen ein schonender Sitz des Kapselspannrings im Kapselsack möglich ist. Es ist möglich, dass eine oder beide der Kammern nur abschnittsweise entlang des Umfangs des Kapselspannrings verlaufen. Bevorzugt wird jedoch, dass zumindest eine, bevorzugt beide, der Kammern sich über einen vollständigen Umfang des Kapselspannrings erstreckt bzw. erstrecken.

Beide Kammern sind grundsätzlich dazu ausgestaltet, je ein Fluid aufzunehmen. Dies bedeutet, dass jede der Kammern ein Fluid, wie beispielsweise eine Flüssigkeit, aufnehmen und bevorzugt auch in sich halten kann. Es gibt jedoch Ausführungsformen wo eine oder beide Kammern mit Öffnungen oder Poren versehen sind, die ein gezieltes Austreten oder Eintreten von Fluid aus der Kammer oder in die Kammer ermöglichen sollen. Auch in diesen Ausführungsformen definieren die Kammern jedoch einen Hohlraum in ihrem Inneren, der durch die Hülle der Kammern abgeschlossen ist, auch wenn diese Hülle u.U. perforiert ist.

Durch die beiden Kammern des Kapselspannrings ist es einerseits möglich, die Intraokularlinse sicher in einer Nut zwischen den beiden Kammern zu fixieren, und andererseits eine besonders ausgeprägte Flexibilität des Kapselspannrings zu ermöglichen. Beispielsweise können die Kammern dazu eingesetzt werden, den Kapselspannring zu stabilisieren, erst nachdem er implantiert wurde. Entsprechend können die Kammern auch dazu verwendet werden, einen bereits implantierten Kapselspannring zu destabilisieren, um ein Entfernen des Kapselspannrings aus dem Kapselsack zu erleichtern. Mit anderen Worten ermöglicht die erfindungsgemäße Ausgestaltung des Kapselspannrings ein Erhöhen oder Reduzieren der Stabilität des Rings zum Zwecke der Implantation, des Einbringens in den Augenkapselsack und des Herausnehmens aus dem Augenkapselsack. Bevorzugt ist der Kapselspannring aus biokompatiblem, sterilisierbarem Material, insbesondere aus Hydroxyethylmethacrylat-Co-Methylmehtacrylat (HEMA/MMA), Silikon, Latex, Kollagen und/oder Hydrogel, ausgebildet. Diese Materialien haben sich hinsichtlich ihrer Verarbeitbarkeit und ihrer Verträglichkeit im Kapselsack eines Patienten als besonders geeignet erwiesen. Besonders vorteilhaft ist an diesen Materialien, dass sie in weiten Grenzen elastisch und/oder weich sind, sodass der Kapselspannring mit geringem Kraftaufwand verformt werden kann. Es sind jedoch auch andere Materialien grundsätzlich einsetzbar, sofern sie über eine gewisse Verformbarkeit verfügen, die von den operativen Bedingungen abhängt, unter denen sie eingesetzt werden sollen. Hierunter fallen insbesondere die Größe des Kapselsacks und die Zugänglichkeit des Kapselsacks von außen sowie die verfügbare Fläche für einen Einschnitt in die Hülle des Kapselsacks.

Erfindungsgemäß sind die zwei Kammern durch zwei nahtlos miteinander zusammengefügte ringförmige Schläuche ausgebildet. Unter ringförmigen Schläuchen wird in diesem Zusammenhang verstanden, dass die Schläuche selbst einen Ring bilden, indem sie endlos ausgebildet sind. Dabei können die Schläuche jeweils eine Torusform bilden, die auch als Reifenform oder Donutform bezeichnet wird. Im Querschnitt senkrecht zur Ringebene können die Schläuche sowohl kreisförmig sein als auch oval oder polygonal. Bevorzugt wird die Kreisform des Querschnitts der Schläuche in dieser Ebene, wobei sich die Form des Querschnitts je nach Inhalt der entsprechenden Kammer verändern kann. Beispielsweise kann beim Einfüllen eines Fluids unter hohem Druck ein kreisförmiger Querschnitt sichergestellt werden, während der Querschnitt nach dem Einfüllen eines Fluids unter niedrigerem Druck leicht ovale oder polygonale Formen annehmen kann.

Eine "nahtlose Verbindung" der Schläuche meint, dass die Schläuche unmittelbar miteinander verbunden sind, ohne dass hierbei eine nach außen hervorstehende Wulst als Naht gebildet wird. Eine solche Naht hätte Nachteile im Hinblick auf die Fixierung der Intraokularlinse an dem Kapselspannring und soll daher verhindert werden.

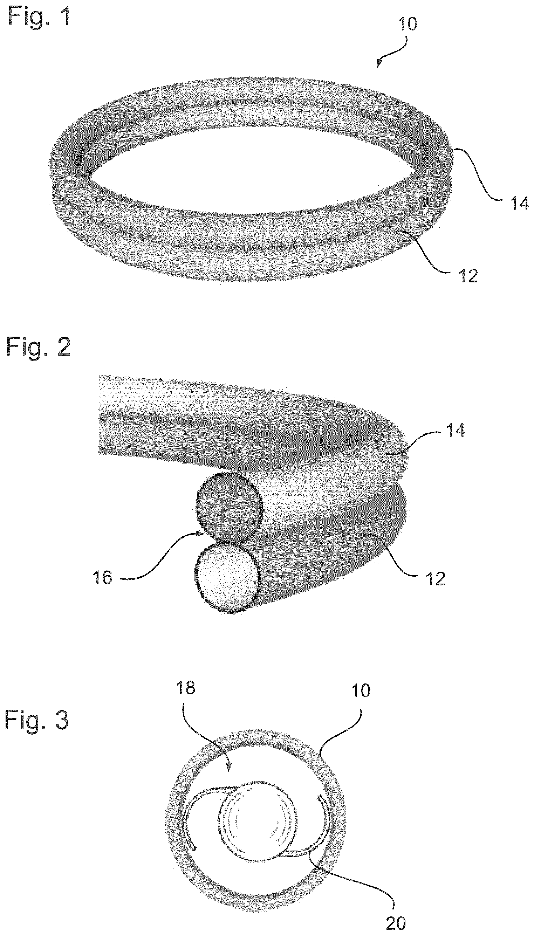

Bevorzugt weisen die zwei Kammern jeweils eine Hülle und einen durch die Hülle umschlossenen Raum auf, wobei der durch die Hülle umschlossene Raum jeder der zwei Kammern jeweils einen konvexen, insbesondere einen kreisförmigen oder ovalförmigen, Querschnitt quer zur Umfangsrichtung aufweist. Das bedeutet, dass die Querschnitte der beiden Kammern zumindest überwiegend so ausgebildet sind, dass eine Verbindung zwischen zwei beliebigen Punkten der Hülle zumindest mehrheitlich, bevorzugt vollständig durch den von der Hülle umschlossenen Raum verläuft. Diese Anordnung ist in Figuren 1 und 2 beispielhaft wiedergegeben.

Der kreisförmige oder ovalförmige Querschnitt sind idealisierte Beschreibungen solcher Querschnitte, die in der Praxis auch deformiert oder mit Spitzen und Ecken versehen sein können, insbesondere um der Anatomie eines Patienten oder einer Intraokularlinse angepasst zu sein.

Mit Vorteil bilden die zwei Kammern zwei parallel zu einer ebenen, gewölbten oder gefalteten Fläche angeordnete Ringe, die in senkrecht zu der Fläche verlaufender Richtung einander kontaktieren. Hierdurch wird ein Doppelring gebildet, wobei die den Doppelring bildenden Ringe bevorzugt torusförmig sind und ihre Mittelpunkte auf einer gemeinsamen, senkrecht zu den Radien der Tori verlaufenden Achse liegen. Diese Anordnung ist in Figuren 1 und 2 beispielhaft wiedergegeben.

Dabei wird bevorzugt, dass die zwei Kammern einander entlang eines Rings oder Teilrings kontaktieren und somit die Nut bilden.

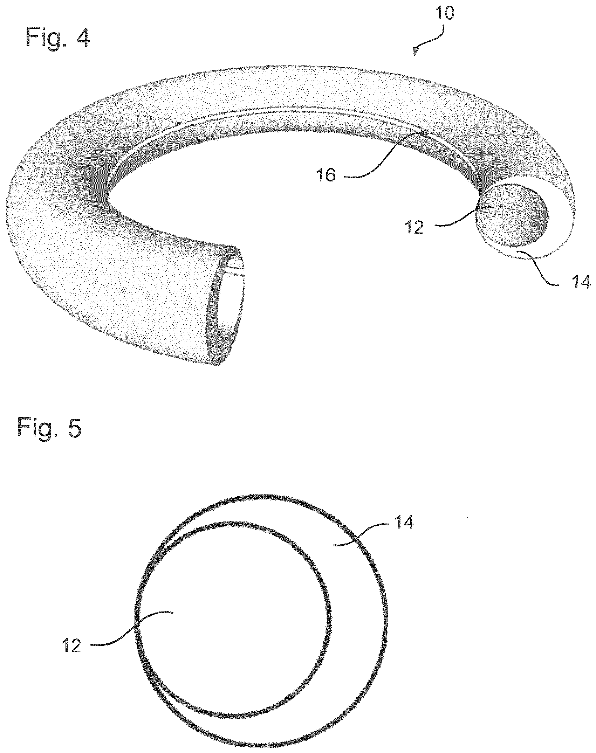

Alternativ wird bevorzugt, dass der durch die Hülle umschlossene Raum einer ersten der zwei Kammern einen konvexen, insbesondere einen kreisförmigen oder ovalförmigen, Querschnitt quer zur Umfangsrichtung aufweist und der durch die Hülle umschlossene Raum einer zweiten der zwei Kammern einen konkav-konvexen, insbesondere einen sichelförmigen, Querschnitt quer zur Umfangsrichtung aufweist, so dass die erste Kammer innerhalb des Außenumfangs der zweiten Kammer angeordnet ist. Das bedeutet, dass der Querschnitt der ersten Kammer zumindest überwiegend so ausgebildet ist, dass eine Verbindung zwischen zwei beliebigen Punkten der Hülle zumindest mehrheitlich, bevorzugt vollständig durch den von der Hülle umschlossenen Raum verläuft. Der Querschnitt der zweiten Kammer ist hingegen zumindest überwiegend so ausgebildet dass eine Verbindung zwischen zwei Punkten für eine Mehrheit der möglichen Punkte auf der Hülle zumindest teilweise außerhalb des umschlossenen Raums verläuft und nur für eine Minderheit der möglichen Punkte vollständig innerhalb des umschlossenen Raums verläuft. Diese Anordnung ist beispielhaft in Figur 4 und 5 wiedergegeben.

Auch in dieser Ausführungsform sind der kreisförmige oder ovalförmige Querschnitt idealisierte Beschreibungen solcher Querschnitte, die in der Praxis auch deformiert oder mit Spitzen und Ecken versehen sein können, insbesondere um der Anatomie eines Patienten oder einer Intraokularlinse angepasst zu sein.

Bevorzugt bilden die zwei Kammern zwei parallel zu einer Fläche angeordnete Ringe, die parallel zu der Fläche einander kontaktieren. Mit Vorzug kontaktieren sich die Ringe entlang einer inneren Umfangsfläche des Kapselspannrings, die sie somit bilden und in der die Nut ausgebildet ist.

Mit Vorteil ist eine erste der Kammern dazu ausgebildet, im implantierten Zustand ein Fluid, insbesondere Hyaluron oder Salzlösung, aufzunehmen, um den Kapselspannring in seiner Form zu stabilisieren.

In einer weiteren bevorzugten Ausführungsform hat die erste der Kammern eine selbstexpandierende Struktur, sodass sie durch Perforation ihrer Hülle aus einem durch ein Vakuum zusammengezogenen Zustand in einen expandierten Zustand ausdehnbar ist, um den Kapselspannring in seiner Form zu stabilisieren.

In einer weiteren bevorzugten Ausführungsform kann der Kapselspannring mehr als zwei Kammern aufweisen, wobei er sowohl eine Kammer zur druckdichten Aufnahme eines Fluids zur Stabilisierung als auch eine Kammer mit selbstexpandierender Struktur aufweist, um den Kapselspannring in seiner Form zu stabilisieren. In diesem Fall kann der Kapselspannring auch drei oder mehr verschiedene Kammern haben, wobei die weitere Kammer oder weiteren Kammern einem weiteren Zweck dienen kann oder können.

Mit Vorteil weist eine der Kammern Perforationen auf, um ein im implantierten Zustand in dieser Kammer aufgenommenes Fluid an die Umgebung des Kapselspannrings abzugeben. Vorzugsweise liegen bei dem Kapselspannring zumindest zwei Kammern vor, die zur Aufnahme eines Fluids ausgebildet sind. Denn so kann eine Stabilisierung des Kapselspannrings nach der Implantation und eine Destabilisierung für Einführen oder Herausnehmen effizient umgesetzt werden, indem zumindest eine dieser Kammern mit Druck beaufschlagt oder selbst expandierend ausgebildet werden kann, während eine weitere Kammer der Abgabe von Fluid, insbesondere einem Medikament, dienen kann.

Bei dem in der mit Perforationen versehenen Kammer aufgenommenen Fluid kann es sich insbesondere um ein Medikament handeln, das durch die Perforationen protrahiert an die Umgebung abgegeben werden kann. Hierfür kommen insbesondere solche Substanzen in Frage, die in Kontakt zytotoxisch wirken und die germinative Zone der Kapselsack-Epithelien, die für den regeneratorischen Nachstar verantwortlich ist, in ihrer Proliferationsfähigkeit hemmen. Weitere Beispiele sind allgemein antientzündliche Substanzen, die alternativ oder zusätzlich in der zweiten oder einer weiteren Kammer des Kapselspannrings aufgenommen sein und durch Perforationen an die Umgebung abgegeben werden können. Mit Vorteil weist der Kapselspannring einen Innendurchmesser von 9 mm bis 13 mm und eine radiale Ausdehnung von 0,2 mm bis 0,5 mm auf. Ferner wird bevorzugt, dass der Kapselspannring eine Höhe senkrecht zu einer Ebene des Rings von zwischen 0,5 mm und 0,9 mm aufweist. Dabei ist mit der Ebene des Rings die Ringebene, nämlich diejenige Ebene gemeint, in der sich der Ring, das heißt beispielsweise der Torus, im Wesentlichen erstreckt. In dieser Ebene wird auch der Durchmesser des Rings gemessen, während der Querschnitt der Kammern senkrecht zu dieser Ebene betrachtet wird.

In einer bevorzugten Ausführungsform ist der Kapselspannring mit einer Intraokularlinse verbunden, die an einer Innenseite des Kapselspannrings mit dem Kapselspannring verbunden, insbesondere verklebt oder verschweißt, ist. Es ist also nicht erforderlich, dass der Kapselspannring als separates Element in den Kapselsack implantiert und erst dort mit dem Kapselspannring verbunden wird, sondern es ist möglich, dass die Intraokularlinse bereits vor der Implantation mit dem Kapselspannring verbunden ist. In diesem Fall kann die Verbindung zwischen dem Kapselspannring und der Intraokularlinse leichter und damit auch hochwertiger hergestellt werden, weil es außerhalb des Kapselsacks wesentlich leichter möglich ist, die Intraokularlinse mit dem Kapselspannring zu verbinden.

Eine beispielhafte Anwendung des Kapselspannrings liegt in einem Verfahren zur Implantation des Kapselspannrings in einem Augenkapselsack. Dabei wird der Kapselspannring durch eine wie üblich in den Kapselsack eingebrachte Öffnung in den Kapselsack eingesetzt, während keine der Kammern mit Fluid gefüllt ist. Dies bedeutet, dass die Kammern bevorzugt im Wesentlichen leer sind, sodass der externe Luftdruck die Kammern zusammendrückt, was durch ein geeignet gewähltes Material, beispielsweise Silikon, aber auch eine Vielzahl anderer Materialien, möglich ist. Alternativ ist es auch möglich, dass lediglich eine der beiden Kammern leer ist oder beide Kammern mit einem Medium unter geringem Druck versehen sind. Dies stellt sicher, dass der Kapselspannring für das Einsetzen in den Kapselsack sehr flexibel ist und daher besonders leicht und verletzungsfrei eingesetzt werden kann.

Nach dem Einbringen des Kapselspannrings in den Augenkapselsack kann beispielsweise durch eine Spritze Hyaluron, Salzlösung oder eine andere Flüssigkeit, ein Gel oder ein Gas in eine der Kammern eingebracht werden, um den Kapselspannring hierdurch zu stabilisieren. Durch den erhöhten Innendruck behält der Kapselspannring seine Form im Wesentlichen bei, wie dies beispielsweise von Fahrradschläuchen, Autoreifen oder dergleichen bekannt ist.

Darüber hinaus kann in die zweite der Kammern vorzugsweise ein Medikament oder eine andere Substanz eingebracht werden, die entweder zur weiteren Stabilisierung des Kapselspannrings dienen kann oder beispielsweise durch Mikroperforationen protrahiert an die Umgebung abgegeben werden kann. Insbesondere wenn ein Medikament in die zweite Kammer eingebracht wird, kann ein wirkungsvoller Schutz gegen postoperative Traumata erzielt werden.

Zum Entfernen des erfindungsgemäßen Kapselspannrings ist es möglich, den Inhalt der Kammern beispielsweise mit einer Spritze zu entfernen, wodurch der Kapselspannring wieder sehr flexibel werden kann und daher leicht aus dem Kapselsack entfernbar ist.

Weitere Vorteile und Merkmale der Erfindung ergeben sich aus der Gesamtheit der Patentansprüche sowie der nachfolgenden Figurenbeschreibung.

### KURZE FIGURENBESCHREIBUNG

Fig. 1 zeigt eine räumliche Darstellung eines Kapselspannrings gemäß einer bevorzugten Ausführungsform.
Fig. 2 zeigt eine partielle Schnittansicht des Kapselspannrings aus Fig. 1.
Fig. 3 zeigt eine Draufsicht eines Kapselspannrings mit einer Intraokularlinse gemäß einer beispielhaften Ausführungsform.
Fig. 4 zeigt eine räumliche Darstellung eines Kapselspannrings gemäß einer zweiten bevorzugten Ausführungsform.
Fig. 5 zeigt eine Querschnittsansicht des Kapselspannrings gemäß Fig. 4.

### WEGE ZUR AUSFÜHRUNG DER ERFINDUNG

In der nachfolgenden Figurenbeschreibung und den beiliegenden Zeichnungen werden gleiche Bezugszeichen für gleiche oder einander entsprechende Elemente und Merkmale verwendet. Für die zweite bevorzugte Ausführungsform werden insbesondere die Unterschiede zur ersten bevorzugten Ausführungsform hervorgehoben und einige Gemeinsamkeiten nicht erneut beschrieben, sondern hiermit auf das zur ersten Ausführungsform Vorgetragene verwiesen.

In Fig. 1 ist eine perspektivische Ansicht eines Kapselspannrings 10, wie er gemäß einer ersten Ausführungsform ausgebildet sein kann. Der Kapselspannring 10 weist eine erste Kammer 12 und eine zweite Kammer 14 auf, die sich jeweils entlang einer Umfangsrichtung des Kapselspannrings 10 erstrecken. Die erste Kammer 12 und die zweite Kammer 14 sind jeweils als ringförmig verlaufende Schläuche mit kreisförmigem Querschnitt ausgebildet. Diese Schläuche bilden somit jeweils einen Torus und die beiden Schläuche sind in axialer Richtung in Bezug auf die Ringform der ersten und zweiten Kammer 12, 14, d.h. senkrecht zur Ringebene, nahtlos miteinander verbunden.

In dieser bevorzugten Ausführungsform ist die erste Kammer 12 ein dichter und damit unter Druck stehendes Medium aufnehmender Mikroschlauch beispielswiese aus medizinischem Silikon, während die zweite Kammer 14 ebenfalls ein Mikroschlauch aus medizinischem Silikon ist, der jedoch mit Mikroperforationen versehen ist. Diese Mikroperforationen können bevorzugt gelasert sein, das heißt durch Wechselwirkung mit Laserlicht in den Mikroschlauch eingebracht worden sein.

Wie Fig. 2 zeigt, definieren die erste Kammer 12 und die zweite Kammer 14 durch ihre schlauchförmige Hülle jeweils einen Hohlraum, der zur Aufnahme eines Fluids geeignet und ausgebildet ist. Insbesondere ist die erste Kammer 12 dazu ausgebildet, Hyaluron oder eine Salzlösung aufzunehmen, um die Struktur des Kapselspannrings 10 zu verstärken oder zu stabilisieren. Je nach Innendruck im Innern der ersten Kammer 12 kann die Stabilisierung des Kapselspannrings 10 besonders wirkungsvoll erfolgen, sodass ein im Wesentlichen steifer Kapselspannring resultieren kann. Es ist jedoch auch möglich, situationsbezogen weniger Fluid in der ersten Kammer 12 aufzunehmen, um einen weniger steifen Kapselspannring 10 zu erhalten. Die Stabilität und Steifigkeit des Kapselspannrings 10 kann zusätzlich zum Druck des Fluids im Innern der ersten Kammer 12 auch beispielsweise über die Viskosität des jeweiligen Fluids gesteuert werden, die temperaturabhängig veränderlich sein kann.

Die zweite Kammer 14 ist in der in den Figs. 1 und 2 gezeigten Ausführungsform mit gelaserten Mikroperforationen versehen. Sie dient dazu, ein Medikament in Form einer flüssigen Substanz aufzunehmen, das beispielsweise postoperative Traumata wie Entzündungen und dergleichen oder Schmerzen verhindern oder hemmen kann. Durch die Mikroperforationen der zweiten Kammer 14 kann das Medikament protrahiert, das heißt über einen langen Zeitraum dosiert, an die Umgebung des Kapselspannrings 10 abgeben werden. Unter anderem über die Größe der Mikroperforationen in der zweiten Kapsel 14 kann eingestellt werden, wie schnell die im Innern der zweiten Kammer 14 befindliche Substanz an die Umgebung des Kapselspannrings 10 abgegeben wird.

Alternativ zu der in den Figs. 1 und 2 dargestellten Ausführungsform ist es auch möglich, dass auch die zweite Kammer 14 keine Perforationen aufweist, das heißt insbesondere zur Aufnahme eines unter Druck stehenden Fluids geeignet ist, um dadurch den Kapselspannring 10 weiter zu stabilisieren. Daneben ist es auch möglich, dass auch die erste Kammer 12 mit einer Öffnung oder mit vielen Mikroperforationen versehen ist, wobei in diesem Fall bevorzugt wird, dass die erste Kammer 12 eine selbstexpandierende Struktur aufweist, so dass sie von sich aus eine stabile Form einnimmt, sobald das Loch oder die Perforationen geöffnet sind und ein Fluid in das Innere der Kammer 12 einströmen kann, um diese sich expandieren zu lassen.

Zwischen der ersten Kammer 12 und der zweiten Kammer 14 ist in der in Fig. 2 gezeigten Darstellung eine Nut 16 zu erkennen. Diese Nut 16 dient dazu, eine intraokulare Linse zu fixieren, wie beispielsweise in Fig. 3 angedeutet ist.

Die Ausführungsform gemäß Fig. 1 und 2 ist so gestaltet, dass die beiden Kammern 12, 14 zumindest an der Innenseite des Kapselspannrings nahtlos miteinander verbunden sind. Dies kann auf verschiedene Weisen erfolgen, die aus dem Stand der Technik grundsätzlich bekannt sind. Durch die insbesondere in Fig. 2 gut zu erkennende Formgebung des Kapselspannrings 10 wird für die Nut 16 ein im Wesentlichen V-förmiger Querschnitt definiert. Zwar sind auch rechteckige oder andere polygonale Querschnitte der Nut 16 für die vorliegende Erfindung grundsätzlich geeignet. Demgegenüber ist aber eine V-förmige Ausgestaltung dieser Nut wie sie insbesondere in Fig. 2 gezeigt ist, besonders vorteilhaft. Denn bei einer solchen Ausgestaltung der Nut 16 lässt sich die Linse sehr leicht zentrieren. Dies fördert eine sichere Aufnahme der Intraokularlinse, weil diese in einem stabilen Gleichgewicht in der Nut 16 gehalten werden kann.

Fig. 3 zeigt eine Draufsicht einer beispielhaften Kombination aus Kapselspannring 10 und Intraokularlinse 18. Die Intraokularlinse 18 umfasst zwei Haptiken 20, wie sie im Bereich der Intraokularlinsen üblich sind. Durch diese Haptiken 20 kann die Intraokularlinse 18 sicher in der Nut 16 des Kapselspannrings 10 gehalten werden.

Falls der Kapselspannring 10 einmal aus dem Kapselsack entnommen werden soll, kann beispielsweise durch eine Spritze oder dergleichen das Fluid aus dem Innern der ersten Kammer 12 und/oder der zweiten Kammer 14 entnommen werden, sodass die Stabilität des Kapselspannrings 10 erheblich nachlässt oder, mit anderen Worten, die Flexibilität des Kapselspannrings 10 erheblich erhöht wird. Dadurch wird der Kapselspannring 10 sehr gut verformbar und kann auch durch sehr kleine Einschnitte in der äußeren Hülle des Kapselsacks entnommen und ggf. durch einen neuen Kapselspannring ersetzt werden.

Fig. 4 illustriert eine zweite bevorzugte Ausführungsform des Kapselspannrings 10. Im Gegensatz zur in Fig. 1 und Fig. 2 gezeigten Anordnung der beiden Kammern 12, 14 sind die beiden Kammern 12, 14 in der zweiten Ausführungsform ineinander angeordnet. Wie sich in Fig. 4 erkennen lässt, wird die erste Kammer 12 von der zweiten Kammer 14 umgeben. Die zweite Kammer 14 bildet einen sichelförmigen Querschnitt, wie deutlich in Fig. 5 zu erkennen ist, und umgibt die erste Kammer 12 mehrheitlich. Die erste Kammer 12 und die zweite Kammer 14 kontaktieren einander entlang einer Innenseite des Kapselspannrings 10, entlang der auch die Nut 16 ausgebildet ist.

Die erste, innenliegende Kammer 12 ist insbesondere ein dichter und damit unter Druck stehendes Medium aufnehmender Mikroschlauch beispielswiese aus medizinischem Silikon, während die zweite Kammer 14 ebenfalls ein Mikroschlauch aus medizinischem Silikon ist, der jedoch mit Mikroperforationen versehen ist. Diese Mikroperforationen können bevorzugt gelasert sein, das heißt durch Wechselwirkung mit Laserlicht in den Mikroschlauch eingebracht worden sein.

Durch die Mikroperforationen kann ein in der zweiten Kammer 14 eingebrachter Wirkstoff retardiert, d.h. nach vollendeter Implantation, an seine Umgebung abgegeben werden, während ein in der ersten Kammer 12 aufgenommenes Medium die Stabilität des Kapselspannrings 10 aufrechterhalten kann.

Insbesondere für die zweite Ausführungsform wird bevorzugt, dass die Intraokularlinse 18 bereits mit dem Kapselspannring 10 verbunden, insbesondere verklebt oder verschweißt ist.

Die vorliegenden Ausführungsformen sind besonders bevorzugte beispielhafte Darstellungen eines Kapselspannrings, der es ermöglicht, sowohl einen Kapselsack sicher daran zu hindern, zusammenzufallen, als auch eine intraokulare Linse sicher und positionsgenau positioniert zu halten und dabei besonders gut implantierbar und wieder extrahierbar zu sein.

## Patentansprüche

1. Kapselspannring (10) zum Implantieren in einem Augenkapselsack,
wobei der Kapselspannring (10) zwei Kammern (12, 14) aufweist, die durch zwei nahtlos miteinander zusammengefügte ringförmige Schläuche ausgebildet sind und die sich jeweils entlang einer Umfangsrichtung des Kapselspannrings (10) erstrecken,
wobei die Kammern (12, 14) dazu ausgebildet sind, je ein Fluid aufzunehmen, und
wobei der Kapselspannring (10) eine sich entlang der Umfangsrichtung erstreckende Nut (16) aufweist, um dadurch eine Intraokularlinse (18) zu fixieren.

2. Kapselspannring (10) nach Anspruch 1, wobei der Kapselspannring (10) aus biokompatiblem, sterilisierbarem Material, insbesondere aus Hydroxyethylmethacrylat-Co-Methylmethacrylat (HEMA/MMA), Silikon, Latex, Kollagen und/oder Hydrogel, ausgebildet ist.

3. Kapselspannring (10) nach Anspruch 1 oder 2, wobei die zwei Kammern (12, 14) jeweils eine Hülle und einen durch die Hülle umschlossenen Raum aufweisen, wobei der durch die Hülle umschlossene Raum jeder der zwei Kammern (12, 14) jeweils einen konvexen, insbesondere einen kreisförmigen oder ovalförmigen, Querschnitt quer zur Umfangsrichtung aufweist.

4. Kapselspannring (10) nach einem der Ansprüche 1 bis 3, wobei die zwei Kammern (12, 14) zwei parallel zu einer Fläche angeordnete Ringe bilden, die in senkrecht zu der Fläche verlaufender Richtung einander kontaktieren.

5. Kapselspannring (10) nach Anspruch 3 oder 4, wobei die zwei Kammern (12, 14) einander entlang eines Rings oder Teilrings kontaktieren und somit die Nut (16) bilden.

6. Kapselspannring (10) nach Anspruch 1 oder 2, wobei die zwei Kammern (12, 14) jeweils eine Hülle und einen durch die Hülle umschlossenen Raum aufweisen,
wobei der durch die Hülle umschlossene Raum einer ersten (12) der zwei Kammern (12, 14) einen konvexen, insbesondere einen kreisförmigen oder ovalförmigen, Querschnitt quer zur Umfangsrichtung aufweist
und wobei der durch die Hülle umschlossene Raum einer zweiten (14) der zwei Kammern (12, 14) einen konkav-konvexen, insbesondere einen sichelförmigen, Querschnitt quer zur Umfangsrichtung aufweist,
so dass die erste Kammer (12) innerhalb des Außenumfangs der zweiten Kammer (14) angeordnet ist.

7. Kapselspannring (10) nach einem der Ansprüche 1, 2 und 6, wobei die zwei Kammern (12, 14) zwei parallel zu einer Fläche angeordnete Ringe bilden, die parallel zu der Fläche einander kontaktieren.

8. Kapselspannring nach einem der Ansprüche 6 und 7, wobei die zwei Kammern (12, 14) gemeinsam eine Innenseite des Kapselspannrings (10) bilden, in der die Nut (16) ausgebildet ist.

9. Kapselspannring (10) nach einem der Ansprüche 1 bis 8, wobei eine erste (12) der zwei Kammern (12, 14) dazu ausgebildet ist, im implantierten Zustand ein Fluid, insbesondere Hyaluron oder Salzlösung, aufzunehmen, um den Kapselspannring (10) in seiner Form zu stabilisieren.

10. Kapselspannring (10) nach einem der Ansprüche 1 bis 8, wobei eine erste (12) der zwei Kammern (12, 14) eine selbstexpandierende Struktur hat, so dass sie durch Perforation ihrer Hülle aus einem durch ein Vakuum zusammengezogenen Zustand in einen expandierten Zustand ausdehnbar ist, um den Kapselspannring (10) in seiner Form zu stabilisieren.

11. Kapselspannring (10) nach einem der vorhergehenden Ansprüche, wobei eine zweite (14) der zwei Kammern (12, 14) Perforationen aufweist, um ein im implantierten Zustand in der zweiten Kammer (14) aufgenommenes Fluid an die Umgebung des Kapselspannrings (10) abzugeben.

12. Kapselspannring (10) nach einem der vorhergehenden Ansprüche, wobei der Kapselspannring (10) einen Innendurchmesser von 9 mm bis 13 mm und eine radiale Ausdehnung von 0,2 mm bis 0,5 mm aufweist.

13. Kapselspannring (10) nach einem der vorhergehenden Ansprüche, wobei der Kapselspannring (10) eine Höhe senkrecht zu einer Ebene des Rings von zwischen 0,5 mm und 0,9 mm aufweist.

14. Kapselspannring (10) nach einem der vorhergehenden Ansprüche mit einer Intraokularlinse (18), die an einer Innenseite des Kapselspannrings (10) mit dem Kapselspannring (10) verbunden, insbesondere verklebt oder verschweißt, ist.

## Claims

1. Capsular tension ring (10) for implantation in the capsular bag of an eye,
wherein the capsular tension ring (10) has two chambers (12, 14) which are formed by two annular tubes which are joined together seamlessly and which each extend in a circumferential direction of the capsular tension ring (10),
wherein the chambers (12, 14) are each adapted to receive a fluid, and
wherein the capsular tension ring (10) has a groove (16) extending in the circumferential direction (16) in order thereby to fix an intraocular lens (18) in position.

2. Capsular tension ring (10) according to claim 1, wherein the capsular tension ring (10) is formed from biocompatible, sterilisable material, and in particular from hydroxyethyl methacrylate-methyl methacrylate (HEMA-MMA) copolymer, silicone, latex, collagen and/or hydrogel.

3. Capsular tension ring (10) according to claim 1 or 2, wherein the two chambers (12, 14) each have an envelope and a space enclosed by the envelope, the space enclosed by the envelope of each of the two chambers (12, 14) being, in each case, of a convex, and in particular of a circular or oval, cross-section transversely to the circumferential direction.

4. Capsular tension ring (10) according to one of claims 1 to 3, wherein the two chambers (12, 14) form two rings arranged parallel to a surface, which come into contact with one another in a direction lying perpendicular to the surface.

5. Capsular tension ring (10) according to claim 3 or 4, wherein the two chambers (12, 14) come into contact with one another along a ring or part-ring and thus form the groove (16).

6. Capsular tension ring (10) according to claim 1 or 2, wherein the two chambers (12, 14) each have an envelope and a space enclosed by the envelope,
wherein the space enclosed by the envelope of a first one (12) of the two chambers (12, 14) is of a convex, and in particular of a circular or oval, cross-section transversely to the circumferential direction,
and wherein the space enclosed by the envelope of a second one (14) of the two chambers (12, 14) is of a concave-convex, and in particular of a crescent-shaped, cross-section transversely to the circumferential direction,
the first chamber (12) thus being arranged inside the outer circumference of the second chamber (14).

7. Capsular tension ring (10) according to one of claims 1, 2 and 6, wherein the two chambers (12, 14) form two rings arranged parallel to a surface, which make contact with one another parallel to the surface.

8. Capsular tension ring according to either of claims 6 and 7, wherein the two chambers (12, 14) together form an inner side of the capsular tension ring (10), in which the groove (16) is formed.

9. Capsular tension ring (10) according to one of claims 1 to 8, wherein a first one (12) of the two chambers (12, 14) is adapted to receive a fluid, and in particular hyaluronic acid or a salt solution, in the implanted state, to hold the capsular tension ring (10) stable in its shape.

10. Capsular tension ring (10) according to one of claims 1 to 8, wherein a first one (12) of the two chambers (12, 14) has a self-expanding structure, it thus being able to be expanded, by perforation of its envelope, from a state in which it is collapsed by a vacuum to an expanded state, in order to hold the capsular tension ring (10) stable in its shape.

11. Capsular tension ring (10) according to one of the preceding claims, wherein a second one (14) of the two chambers (12, 14) has perforations in order, in the implanted state, to dispense a fluid held in the second chamber (14) into the surroundings of the capsular tension ring (10).

12. Capsular tension ring (10) according to one of the preceding claims, wherein the capsular tension ring (10) has an inside diameter of 9 mm to 13 mm and a radial extent of 0.2 mm to 0.5 mm.

13. Capsular tension ring (10) according to one of the preceding claims, wherein the capsular tension ring (10) has a height perpendicular to a plane of the ring of between 0.5 mm and 0.9 mm.

14. Capsular tension ring (10) according to one of the preceding claims having an intraocular lens (18) which is connected, and in particular is bonded or welded, to the capsular tension ring (10) at an inner side of the capsular tension ring (10).

## Revendications

1. Anneau de tension capsulaire (10) destiné à être implanté dans un sac capsulaire oculaire,
dans lequel l'anneau de tension capsulaire (10) présente deux chambres (12, 14) qui sont réalisées par deux tuyaux annulaires réunis sans soudure et qui s'étendent chacun le long d'une direction circonférentielle de l'anneau de tension capsulaire (10),
dans lequel les chambres (12, 14) sont réalisées pour recevoir chacune un fluide, et
dans lequel l'anneau de tension capsulaire (10) présente une rainure (16) s'étendant le long de la direction circonférentielle pour fixer ainsi une lentille intraoculaire (18).

2. Anneau de tension capsulaire (10) selon la revendication 1, dans lequel l'anneau de tension capsulaire (10) est réalisé en un matériau biocompatible stérilisable, en particulier en hydroxyéthylméthacrylate-co-méthylméthacrylate (HEMA/MMA), silicone, latex, collagène et/ou hydrogel.

3. Anneau de tension capsulaire (10) selon la revendication 1 ou 2, dans lequel les deux chambres (12, 14) présentent chacune une enveloppe et un espace entouré par l'enveloppe, dans lequel l'espace entouré par l'enveloppe de chacune des deux chambres (12, 14) présente à chaque fois une section transversale convexe, en particulier circulaire ou ovale, transversalement par rapport à la direction circonférentielle.

4. Anneau de tension capsulaire (10) selon l'une quelconque des revendications 1 à 3, dans lequel les deux chambres (12, 14) forment deux anneaux qui sont agencés parallèlement à une surface et qui sont en contact l'un avec l'autre dans une direction s'étendant perpendiculairement à la surface.

5. Anneau de tension capsulaire (10) selon la revendication 3 ou 4, dans lequel les deux chambres (12, 14) sont en contact l'une avec l'autre le long d'un anneau ou d'un anneau partiel et forment ainsi la rainure (16).

6. Anneau de tension capsulaire (10) selon la revendication 1 ou 2, dans lequel les deux chambres (12, 14) présentent chacune une enveloppe et un espace entouré par l'enveloppe,
dans lequel l'espace entouré par l'enveloppe d'une première (12) des deux chambres (12, 14) présente à chaque fois une section transversale convexe, en particulier circulaire ou ovale, transversalement par rapport à la direction circonférentielle
et dans lequel l'espace entouré par l'enveloppe d'une seconde (14) des deux chambres (12, 14) présente à chaque fois une section transversale concave-convexe, en particulier en forme de croissant, transversalement par rapport à la direction circonférentielle,
de sorte que la première chambre (12) est agencée à l'intérieur de la périphérie extérieure de la seconde chambre (14).

7. Anneau de tension capsulaire (10) selon l'une quelconque des revendications 1, 2 et 6, dans lequel les deux chambres (12, 14) forment deux anneaux qui sont agencés parallèlement à une surface et qui sont en contact l'un avec l'autre parallèlement à la surface.

8. Anneau de tension capsulaire selon l'une quelconque des revendications 6 et 7, dans lequel les deux chambres (12, 14) forment ensemble un côté intérieur de l'anneau de tension capsulaire (10), côté intérieur dans lequel la rainure (16) est réalisée.

9. Anneau de tension capsulaire (10) selon l'une quelconque des revendications 1 à 8, dans lequel une première (12) des deux chambres (12, 14) est réalisée pour, à l'état implanté, loger un fluide, en particulier une solution hyaluronique ou saline, afin de stabiliser l'anneau de tension capsulaire (10) quant à sa forme.

10. Anneau de tension capsulaire (10) selon l'une quelconque des revendications 1 à 8, dans lequel une première (12) des deux chambres (12, 14) a une structure auto-expansive de sorte que, par perforation de son enveloppe, elle peut se dilater d'un état contracté par un vide à un état dilaté afin de stabiliser l'anneau de tension capsulaire (10) quant à sa forme.

11. Anneau de tension capsulaire (10) selon l'une quelconque des revendications précédentes, dans lequel une seconde (14) des deux chambres (12, 14) présente des perforations pour délivrer dans l'environnement de l'anneau de tension capsulaire (10) un fluide logé dans la seconde chambre (14) à l'état implanté.

12. Anneau de tension capsulaire (10) selon l'une quelconque des revendications précédentes, dans lequel l'anneau de tension capsulaire (10) présente un diamètre intérieur de 9 mm à 13 mm et une dimension radiale de 0,2 mm à 0,5 mm.

13. Anneau de tension capsulaire (10) selon l'une quelconque des revendications précédentes, dans lequel l'anneau de tension capsulaire (10) présente une hauteur, perpendiculairement à un plan de l'anneau, comprise entre 0,5 mm et 0,9 mm.

14. Anneau de tension capsulaire (10) selon l'une quelconque des revendications précédentes avec une lentille intraoculaire (18) qui est assemblée, en particulier collée ou soudée, à l'anneau de tension capsulaire (10) au niveau d'un côté intérieur de l'anneau de tension capsulaire (10).
